(19) Europäisches Patentamt
European Patent Office
Office européen des brevets

(11) EP 1 471 150 A2

(12) # DEMANDE DE BREVET EUROPEEN

(43) Date de publication:
**27.10.2004 Bulletin 2004/44**

(21) Numéro de dépôt: **04003945.5**

(22) Date de dépôt: **18.10.1993**

(51) Int Cl.[7]: **C12P 21/08**, A61K 39/395, G01N 33/53, C12N 1/21, C07K 14/445

(84) Etats contractants désignés:
**AT BE CH DE DK ES FR GB GR IE IT LI LU MC NL PT SE**

(30) Priorité: **19.10.1992 FR 9212488**

(62) Numéro(s) de document de la (des) demande(s) initiale(s) en application de l'article 76 CBE:
**93923584.2 / 0 666 916**

(71) Demandeur: **INSTITUT PASTEUR**
**75724 Paris Cédex 15 (FR)**

(72) Inventeurs:
- **Druilhe, Pierre**
  **75015 Paris (FR)**
- **Bouharoun-Tayoun, Hasnaa,**
  **Université Libanaise**
  **Dekwaneh, Beyrouth (LB)**
- **Oeuvray, Claude**
  **75015 Paris (FR)**

(74) Mandataire: **Desaix, Anne et al**
**Ernest Gutmann - Yves Plasseraud S.A.**
**3, rue Chauveau-Lagarde**
**75008 Paris (FR)**

Remarques:
Cette demande a été déposée le 20 - 02 - 2004 comme demande divisionnaire de la demande mentionnée sous le code INID 62.

(54) **Antigènes de plasmodium falciparum inducteurs d'anticorps protecteurs**

(57) L'invention concerne une molécule, protéine ou peptide, caractérisés en ce qu'ils sont reconnus par des anticorps cytophiles de sujets prémunis contre l'infection par les *Plasmodia* et reconnus par les anticorps non cytophiles de sujets sensibles à l'infection par les Plasmodiae. Ces anticorps sont capables de bloquer la phase de développement érythrocytaire du parasite par coopération avec des cellules accessoires telles que les monocytes.

EP 1 471 150 A2

SEQUENCE LISTING

<110> INSTITUT PASTEUR

<120> ANTIGENES DE PLASMODIUM FALCIPARUM INDUCTEURS D'ANTICORPS PROTECTEURS

<130> JAZ/VG - B1948AB.1

<140> New Divisional Application
<141> 2004-02-13

<150> FR 9212488
<151> 1992-10-19

<150> PCT/FR93/01024
<151> 1993-10-18

<150> EP93923584.2
<151> 1993-10-18

<160> 10

<170> PatentIn version 3.1

<210> 1
<211> 64
<212> PRT
<213> Plasmodium falciparum

<400> 1

```
His Glu Arg Ala Lys Asn Ala Tyr Gln Lys Ala Asn Gln Ala Val Leu
1               5                   10                  15

Lys Ala Lys Glu Ala Ser Ser Tyr Asp Tyr Ile Leu Gly Trp Glu Phe
            20                  25                  30

Gly Gly Gly Val Pro Glu His Lys Lys Glu Glu Asn Met Leu Ser His
        35                  40                  45

Leu Tyr Val Ser Ser Lys Asp Lys Glu Asn Ile Ser Lys Glu Asn Glu
    50                  55                  60
```

<210> 2
<211> 23
<212> PRT
<213> Plasmodium falciparum

<400> 2

```
His Glu Arg Ala Lys Asn Ala Tyr Gln Lys Ala Asn Gln Ala Val Leu
1               5                   10                  15

Lys Glu Ala Ser Ser Tyr Asp
            20
```

<210> 3
<211> 27
<212> PRT
<213> Plasmodium falciparum

<400> 3

B1948AB.1 EP.ST25.txt

```
Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn
            20                  25


<210>  4
<211>  28
<212>  PRT
<213>  Plasmodium falciparum

<400>  4

Pro Glu His Lys Lys Glu Glu Asn Met Leu Ser His Leu Tyr Val Ser
1               5                   10                  15

Ser Lys Asp Lys Glu Asn Ile Ser Lys Glu Asn Glu
            20                  25


<210>  5
<211>  192
<212>  DNA
<213>  Plasmodium falciparum

<400>  5
catgaaaggg caaaaaatgc ttatcaaaaa gcaaaccaag ctgttttaaa agcaaaagaa      60

gcttctagtt atgattatat tttaggttgg gaatttggag gaggcgttcc agaacacaaa     120

aaagaagaaa atatgttatc acatttatat gtttcttcaa aggataagga aaatatatct     180

aaggaaaatg ag                                                          192


<210>  6
<211>  75
<212>  DNA
<213>  Plasmodium falciparum

<400>  6
catgaaaggg caaaaaatgc ttatcaaaaa gcaaaccaag ctgttttaaa agcaaaagaa      60

gcttctagtt atgat                                                        75


<210>  7
<211>  81
<212>  DNA
<213>  Plasmodium falciparum

<400>  7
gcaaaagaag cttctagtta tgattatatt ttaggttggg aatttggagg aggcgttcca      60

gaacacaaaa aagaagaaaa t                                                 81


<210>  8
<211>  84
<212>  DNA
<213>  Plasmodium falciparum

<400>  8
ccagaacaca aaaaagaaga aaatatgtta tcacatttat atgtttcttc aaaggataag      60
```

B1948AB.1 EP.ST25.txt

```
gaaaatatat ctaaggaaaa tgag                                               84

<210>  9
<211>  21
<212>  DNA
<213>  Séquence artificielle

<220>
<221>  misc_feature
<222>  (1)..(21)
<223>  ADN synthétique


<400>  9
gaaagggcaa aaaatgctta t                                                  21


<210>  10
<211>  21
<212>  DNA
<213>  Séquence artificielle

<220>
<221>  misc_feature
<222>  (1)..(21)
<223>  ADN synthétique


<400>  10
taaaaggaat ctatataaaa g                                                  21
```

## Description

La présente invention a pour objet de nouvelles préparations pour un vaccin antiplasmodial à large spectre.

L'invention a également pour objet un antigène vaccinant de Plasmodium falciparum capable d'induire une résistance au parasite, laquelle résistance reproduit celle observée dans le mécanisme d'immunité protectrice ou prémunition.

L'invention a également pour objet des préparations d'anticorps monoclonaux ou polyclonaux, ou de fragments, chimères obtenus à partir de ces anticorps spécifiques de ces antigènes et susceptibles d'entrer dans une composition pour immunothérapie passive.

Enfin, l'invention a pour objet un kit ou trousse permettant le diagnostic in vitro de l'infection d'un individu par un large spectre de souches de plasmodium.

Les parasites responsables du paludisme chez l'homme, dont notamment Plasmodium falciparum ou Plasmodium vivax pour ne citer que les principaux d'entre eux, présentent chez l'hôte humain des morphologies différentes et expriment des antigènes différents en fonction de leur localisation dans l'organisme de l'hôte infecté. Les différences morphologiques et antigéniques de ces parasites au cours de leur cycle de vie chez l'homme, permettent de définir au moins quatre stades de développement distincts.

Le tout premier stade de développement du parasite chez l'homme correspond à la forme sporozoïte introduite dans le sang de l'hôte, par piqûres d'insectes porteurs du parasite. Le second stade correspond au passage du parasite dans le foie et à l'infection des cellules hépatiques dans lesquelles les parasites se développent pour former les schizontes hépatiques qui, lorsqu'ils sont matures (par exemple pour P. falciparum le 6è jour après pénétration des sporozoïtes), libèrent par éclatement des mérozoites hépatiques. Le troisième stade est caractérisé par l'infection des erythrocytes sanguins par les formes asexuées (mérozoïtes) du parasite; ce stade érythrocytaire de développement correspond à la phase pathogène de la maladie. Le quatrième stade correspond à la formation des formes à potentiel sexué (ou gamétocytes) qui deviendront des formes sexuées ou gamètes extra-cellulaires chez le moustique.

On sait que de nombreuses études ont été entreprises pour isoler à partir des souches de parasites infectantes pour un hôte humain des fractions polypeptidiques, d'une part pour assurer le diagnostic in vitro du paludisme par détection des anticorps correspondants, et d'autre part, pour tenter de vacciner contre le paludisme.

En 1976 fut mis au point l'entretien (tant attendu) de P. falciparum en culture continue dans les GR humains (Trager et Jensen, Science 1976, 193:673; Haynes et coll. 1976). Cette performance facilita considérablement l'accès au parasite et stimula la recherche qui depuis, connaît un essor considérable. Les efforts sont principalement orientés vers la mise au point d'un vaccin désormais nécessaire pour contrôler le paludisme dont l'incidence s'aggrave dans la mesure où la résistance du parasite aux drogues s'étend dans différentes parties du monde.

Dans la recherche d'un vaccin contre l'agent du paludisme, les biologistes sont confrontés à divers problèmes non observés avec d'autres agents infectieux tels que les virus ou les bactéries. Parmi ces difficultés particulières au parasite, nous citons principalement :

- La complexité du cycle biologique du plasmodium, se déroulant chez 2 hôtes différents, le moustique et l'homme, subissant chez l'un une reproduction sexuée, et chez l'autre 2 phases différentes de multiplication asexuée. Ainsi prennent place chez l'homme 2 stades se distinguant par leur site de développement (le foie et la circulation sanguine) et par leurs spécificités antigéniques.
- La diversité antigénique du parasite. Depuis 1983 les antigènes plasmodiaux sont clonés, leurs séquences nucléotidique et protéique analysées. Cette étude détaillée révèle que plus de 50% des antigènes connus présentent un haut degré de polymorphisme d'une souche à l'autre.
- Sur le plan immunologique, la relation hôte-parasite est très subtile. Elle est comme nous l'avons déjà mentionné, pour un parasite donné, très différente selon l'hôte chez lequel il évolue. Ceci entraîne une difficulté de l'interprétation des résultats obtenus dans les modèles expérimentaux.

Par ailleurs dans l'infection naturelle, on ne voit jamais d'immunité stérilisante, comme celle observée par exemple dans les viroses. Pourtant il existe indiscutablement une immunité acquise mais elle est partielle et labile.

Ainsi donc, la complexité et la diversité du parasite ainsi que la singularité de la réponse immunitaire qu'il suscite sont les raisons majeures de l'absence à ce jour de vaccin anti-paludique.

La voie de recherche la plus souvent empruntée dans l'élaboration d'un vaccin contre le paludisme à P. falciparum consiste donc en l'identification (sur la base des moyens cités plus haut) d'un candidat potentiel, et ensuite l'évaluation de son intérêt soit in vitro en testant les anticorps spécifiques en inhibition de la croissance du parasite ou de certaines de ses propriétés (cytoadhérence, formation de rosettes...), soit in vivo par immunisation de singes souvent avec l'adjuvant complet de Freund. La situation actuelle se résumerait donc à l'existence d'un grand nombre de candidats potentiels caractérisés par leurs propriétés biochimiques, leurs séquences nucléotidique et protéique, leur degré de polymorphisme, leur localisation sur le parasite etc. Néanmoins les chercheurs disposent de moyens limités pour évaluer l'intérêt de leurs candidats : 1) des tests in vitro faisant appel à des mécanismes d'action de l'anticorps peu documentés quant à leur validité in vivo, 2) des vaccinations de primates non humains, et donc l'évaluation de l'effet d'un vaccin sur une infection expérimentale dont les paramètres cliniques, parsitologiques et surtout le type d'immunité qui peut être induite, sont très différents de ceux de l'infection naturelle chez l'homme.

La spécificité étroite de la relation hôte-parasite conduit dans les conditions naturelles, à l'opposé de ce que l'on observe dans les modèles animaux, à un équilibre où le parasite survit en induisant chez son hôte naturel une immunité non stérilisante. Cette chronicité de l'infection parasitaire suggère que la majorité des composants moléculaires du parasite sont sélectionnés de façon à protéger le micro-organisme contre les défenses immunitaires de l'individu infecté, et ce par des moyens d'échappement très divers mais spécifiquement adaptés à l'hôte naturel. Chez l'hôte expérimental, le parasite mal adapté se défend moins bien contre le système immunitaire et la protection contre une seule infection traitée est facile à obtenir, et la vaccination est peut être plus facile à obtenir encore.

Gordon-Thomson, Immunity in Malaria, Trans. Roy. So. Trop. Med. Hyg. XXVI(6) 483-514) conclut clairement que l'immunité contre P. falciparum ne peut être acquise que dans les régions où la transmission est quasi continue tous les ans. Cette "tolérance" au parasitisme requiert, au niveau individuel, une infection ininterrompue pendant environ 15 années parfois 20 et jusqu'à 26 ans dans une étude réalisée au Panama. De ceci résulte une immunité associée à une infection latente nécessaire au maintien de la protection. Sergent (1935) propose le terme de "prémunition" pour définir cet "état particulier de résistance, contemporain de l'infection et cessant avec elle".

Ainsi l'immunité (ou Prémunition) acquise contre P. falciparum par l'homme de zone holo ou hyperendémique se caractérise par:

- un très long délai avant son installation (15 à 20 ans d'infection)
- son incapacité à annuler l'infection, il s'agit d'une immunité non stérilisante.
- sa labilité. En l'absence de toute réinfection (pendant plus d'un an), la prémunition est perdue et le sujet redevient susceptible à la maladie lors d'une nouvelle infection.

Les indications en faveur de l'immunité humorale dans la protection acquise contre le paludisme viennent des premières tentatives de transfert passif de sérum d'un individu en phase "chronique" ayant atteint un état de prémunition (c'est-à-dire présentant des parasites circulants en petit nombre sans aucune manifestation clinique) à un sujet en phase aiguë. L'état de ce dernier s'est trouvé amélioré suite à ce transfert passif (Sotiriades 1917, Essais de sérothérapie dans la malaria Grece Med. XIX: 27-28).

Le rôle des anticorps dans la prémunition est démontré par plusieurs expériences de transfert passif effectuées au début des années 60. Le transfert d'IgG purifiées à partir de sérum d'adultes Africains hyperimmuns guérit des enfants victimes d'une infection aiguë en réduisant sensiblement leur parasitémie (Cohen et coll. 1971, Trans. Roy. Soc. Trop. Med. Hyg. 65(2): 125-135 ; McGregor et coll. 1964, the passive transfert of human material immunity, Am. J. Trop. Med. Hyg. 13: 237-239). Les nouveau-nés sont prémunis jusqu'au troisième mois de leur vie grâce aux anticorps maternels, ceci est prouvé par l'effet bénéfique des IgG de cordon ombilical transférées à des enfants souffrant d'un accès aigu à P. falciparum (Edozien et coll. 1962).

Le développement de l'immunité et son efficacité dans la protection de l'homme contre P. falciparum prouve néanmoins l'existence de molécules parasitaires cibles d'une défense immunitaire efficace.

Des expériences récentes ont permis de montrer que

a) les immunoglobulines G (IgG) d'Africains adultes immuns sont protecteurs par transfert passif chez l'homme impaludé (Sabchareon et al, Amer. J. of Trop. Med. and Hyg., vol. 45, n° 3, Sept. 1991, 297-308),

b) que ces anticorps sont incapables, contrairement à ce qui est considéré comme établi, d'inhiber directement l'invasion des globules rouges par les parasites ; en revanche, ils agissent par un mécanisme d'inhibition cellulaire dépendante d'anticorps (ADCI) dans lequel le monocyte joue le rôle de cellule effectrice (Bouharoun-Tayoun et al, J. exp. Med., vol. 172, Dec. 1990 pp. 1633-1641 ; S.Khusmith et al 1983, Inf. Imm. 41(1): 219 et F. Lunel et al, 1989 Inf. Imm. 57: 2043),

c) Ce mécanisme implique nécessairement des anticorps cytophiles, c'est-à-dire capables de se lier par leur récepteur FC au monocyte ; de fait, il a été observé dans le sérum des sujets protégés, une prévalence d'isotypes cytophiles, IgG1 et IgG3, et chez les sujets non protégés, une prépondérance de classes non cytophiles, IgG2 et/ou IgM (H. BOUHAROUN-TAYOUN et al, 1992, Infection and Immunity, pp. 1473-1481).

Un des buts de la présente invention est la mise au point de polypeptides pour la vaccination chez l'homme contre le paludisme, lesquels polypeptides sont une cible des mécanismes de défense prévalant chez les individus ayant acquis une immunité par exposition prolongée au parasite et leur utilisation dans un vaccin en visant à reproduire le même état de résistance, par le même mécanisme que celui observé dans l'établissement de l'immunité protectrice.

L'invention a également pour objet l'utilisation de ces mêmes polypeptides dans un kit de diagnostic in vitro de l'infection de l'homme par un large spectre de souches de plasmodium.

L'invention concerne plus particulièrement des molécules, ou compositions peptidiques ou polypeptidiques, caractérisées par la présence dans leur structure d'une ou plusieurs séquences peptidiques porteuses d'un ou plusieurs épitopes caractéristiques d'une protéine reconnue par des anticorps de classe cytophile, c'est à dire capable de se fixer sur les récepteurs FcR des monocytes par leur région Fc, et non reconnue par des anticorps non cytophiles et de promouvoir un mécanisme de cytotoxicité-anticorps dépendant (ADCI).

Une protéine de l'invention est une protéine de surface de mérozoïte de poids moléculaire 48.000 (48 Kd), présentant les caractéristiques données plus loin.

Les polypeptides de l'invention ont été obtenus par

- l'identification d'une partie de cette protéine de poids moléculaire 48.000 (48 Kd) de la surface de mérozoite laquelle identification est décrite ci-dessous
- la caractérisation biochimique et immunologique de cette protéine 48 Kd
- le criblage d'une banque génomique du plasmodium pour sa capacité à inhiber le couplage d'un anticorps monoclonal spécifique de type IgM qui a comme caractéristique particulière de bloquer la réaction de type ADCI ("antibody dependant cellular inhibition") induite par les IgG spécifiques du plasma de sujets prémunis
- la caractérisation des protéines synthétisées par les clones sélectionnés
- le séquençage de l'insert du clone sélectionné
- la recherche de l'effet fonctionnel des anticorps correspondant à cette protéine dans les tests décrits.

L'intérêt des protéines et peptides de l'invention et leur stratégie d'obtention sont explicites dans la description ci-dessous.

- **Stratégie de sélection des protéines et peptides**

1- Dans Infection And Immunity (pp. 1473-1481, Avril 1992), les auteurs étudient la distribution isotypique d'individus infectés par le plasmodium présentant des états immunologiques variés. Ils ont ainsi montré que les sujets non protégés ont une composition d'anticorps plasmatiques anti plasmodium très en faveur des isotypes non cytophiles à savoir les IgG2 et les IgM. Dans certains cas, cet équilibre concerne les anticorps contre tous les polypeptides de la malaria détectables par Western blott (technique décrite dans Molecular Cloning, 1989, Sambrook et al.) alors que dans d'autres cas, on a pu mettre en évidence des IgG2 spécifiques d'un polypeptide donné, souvent un polypeptide de 48 Kd apparaissant dans certains isolats sous forme de doublet ou un polypeptide de 80-100 Kd. En revanche, le polypeptide de 48 Kd est toujours reconnu par les isotypes IgG1 et IgG3 cytophiles, chez les adultes ayant acquis une résistance à la maladie, ou état de prémunition.

2- Il a souvent été observé dans des expériences de compétition que les Ig totales purifiées des individus non protégés bloquent la réaction d'ADCI (voir description ci-dessous) induite par les IgG des sujets résistants. Ce résultat suggère que les sujets non protégés ont développé des anticorps dirigés contre les mêmes épitopes que ceux qui sont reconnus par les anticorps protecteurs, mais du fait du caractère non cytophile des IgG2 ou des IgM issus des sujets non protégés, ces anticorps sont incapables de promouvoir l'effet destructeur des monocytes mais par contre sont capables d'entrer en compétition avec des anticorps efficaces en ADCI. Quand un tel effet compétiteur a été identifié en utilisant des sérums humains dans lesquels les anticorps contre la protéine 48 Kd étaient de façon prédominante de l'isotype IgG2, cela clairement met en évidence l'intérêt de cette protéine 48 Kd.

Le test d'ADCI a déjà été décrit dans la publication citée ci-dessus (H. BOUHAROUN-TAYOUN et al, Khusmith et al, Lunel et al). Brièvement, il s'agit d'un test d'inhibition de croissance du parasite par les IgG en présence de monocytes. Les monocytes sont isolés par adhérence sur le plastique (dans une plaque à 96 puits) à partir de la fraction de cellules mononucléées du sang périphérique d'un donneur normal. Une culture synchrone de P. falciparum à 0,5% de parasitémie en forme mûre est rajoutée au monocyte dans un rapport monocytes/globules rouges d'environ 1/200. L'hématocrite étant de 2%, le milieu est supplémenté par le sérum ou les IgG à tester. Les cultures témoin consistent en parasites en présence d'IgG normales, parasites en présence de monocytes et d'IgG normales, parasites en présence des IgG à tester.

Selon le cas, la culture sera arrêtée au bout de 24, 48, 72 ou 96 heures. Dans les deux derniers cas, 50 microlitres de milieu de culture sont ajoutés. La parasitémie finale dans chacun des puits est estimée par comptage de 10.000 globules rouges sur frottis coloré. Les résultats sont présentés sous forme d'index spécifique d'inhibition de croissance (IS) exprimés en pourcentage et calculés comme suit, tenant compte de l'effet inhibiteur éventuel sur la culture des monocytes et/ou des anticorps seuls:

$$IS = 1 - \frac{\%\text{Parasitémie culture + IgG test / \% Parasitémie Monocytes + IgG test}}{\text{\% Parasitémie Monocytes + IgG / \% Parasitémie culture + IgG}} \times 100$$

Cette stratégie de sélection d'une protéine potentiellement vaccinante de 48 Kd selon les critères de reconnaissance par des anticorps cytophiles chez les sujets protégés et non cytophiles chez les sujets non protégés, ainsi que par leur capacité d'induire des anticorps pouvant coopérer avec des monocytes en ADCI nous ont amené à sélectionner cette protéine 48 Kd ou des peptides représentant des régions épitopiques de cette protéine comme candidats potentiellement très intéressants pour induire l'effet immunologique protecteur contre des infections au P. falciparum chez des patients.

L'invention concerne également particulièrement des molécules, ou compositions peptidiques ou polypeptidiques, caractérisées par la présence dans leur structure d'une ou de plusieurs séquences peptidiques porteuses d'un ou plusieurs épitopes caractéristiques de la protéine et répondant aux trois critères :

- reconnaissance par des anticorps de classe cytophile chez les sujets protégés et non cytophiles chez les sujets non protégés
- leur capacité d'induire des anticorps pouvant coopérer avec des monocytes en ADCI
- leur caractère non ou peu polymorphe dans la mesure où l'immunité protectrice peut s'exercer à l'égard d'un grand nombre de souches.

Les molécules de l'invention sont toutes les molécules portant des épitopes, reconnues par des anticorps reconnaissant des épitopes portés par la protéine 48 Kd de la surface du mérozoïte.

Une composition polypeptidique selon l'invention est caractérisée par la présence d'une séquence de 64 acides aminés ou d'une séquence dérivée possédant les mêmes propriétés antigéniques, et dont l'exemple est donné dans la formule I suivante :

**his glu arg ala lys asn ala tyr gln lys**
**ala asn gln ala val leu lys ala lys glu**
**ala ser ser tyr asp tyr ile leu gly trp**
**glu phe gly gly gly val pro glu his lys**
**lys glu glu asn met leu ser his leu tyr**
**val ser ser lys asp lys glu asn ile ser**
**lys glu asn glu**

L'invention concerne en premier lieu des peptides monomères synthétiques comprenant une séquence peptidique unique de 64 acides aminés répondant respectivement à la formule sus-indiquée, et dont les acides aminés terminaux possèdent des extrémités respectivement amine et carboxylique libres, ou des oligomères contenant notamment des multiples de l'une quelconque de la susdite séquence peptidique.

Il va de soi que les fonctions réactives libres que sont susceptibles de posséder certains acides aminés entrant dans la constitution des molécules selon l'invention, notamment les groupes carboxyles libres portés par les groupes Glu et par l'acide aminé C-terminal, d'une part, et/ou les groupes libres portés par l'acide aminé N-terminal ou par des acides aminés intérieurs à la chaîne peptidique, par exemple Lys, d'autre part peuvent être modifiées, dès lors que cette modification n'entraîne pas une modification des propriétés antigéniques, le cas échéant immunogéniques, de l'ensemble de la molécule. Les molécules ainsi modifiées entrent naturellement dans le cadre de la protection donnée à l'invention par les revendications. Ces fonctions carboxyles sont éventuellement acylées ou estérifiées.

D'autres modifications entrent également dans le cadre de l'invention. En particulier, fonctions amine ou ester, ou les deux à la fois, des acides aminés terminaux peuvent être engagées elles-mêmes dans des liaisons avec d'autres acides aminés. Par exemple, l'acide N-terminal peut être lié à une séquence comprenant de 1 à plusieurs acides amines correspondant à une partie de la région C-terminale d'un autre peptide conforme à la définition qui en a été donnée plus haut, ou vice-versa.

Il va de soi également que toute séquence peptidique issue de la modification, par substitution et/ou par addition et/ou suppression d'un ou plusieurs acides aminés, de la séquence peptidique de 64 acides aminés, entre dans le cadre de la protection donnée à l'invention par les revendications, dès lors que cette modification n'altère pas les propriétés antigéniques ou immunogéniques du polypeptide, notamment lorsque ces propriétés immunogéniques ont été renforcées de façon adéquate, par exemple par association de ce polypeptide avec un adjuvant immunologique approprié (par exemple un muramylpeptide) ou par couplage avec une molécule porteuse de poids moléculaire plus élevé (par exemple une sérum-albumine ou une poly-lysine) ou une toxine du type tétanique ou un autre antigène de P. falciparum.

L'invention concerne plus généralement toute molécule caractérisée par la présence dans sa structure d'une ou plusieurs séquences peptidiques présentant des réactions immunologiques croisées avec la séquence peptidique répondant à la formule précédente vis-à-vis des anticorps inductibles par ces dernières in vivo.

L'invention concerne aussi tout peptide dont la structure découle du précédent et notamment de l'un des trois peptides de formule II, III, IV

**his glu arg ala lys asn ala tyr gln lys ala asn** **II**
**gln ala val leu lys glu ala ser ser tyr asp**

**ala lys glu ala ser ser tyr asp tyr ile leu gly** **III**
**trp glu phe gly gly gly val pro glu his lys lys**
**glu glu asn**

**pro glu his lys lys glu glu asn met leu ser his** **IV**
**leu tyr val ser ser lys asp lys glu asn ile ser**
**lys glu asn glu**

Comme dans le cas du premier peptide sus-défini, les différents peptides qui viennent d'être nommés peuvent être modifiés sans pour autant sortir du cadre de l'invention, dès lors que ces modifications de structure n'entraîneraient pas des transformations profondes de leurs propriétés antigéniques.

Les peptides selon l'invention peuvent être préparés par les techniques classiques, dans le domaine de la synthèse des peptides. Cette synthèse peut être réalisée en solution homogène ou en phase solide.

Par exemple, on aura recours à la technique de synthèse en solution homogène décrit par HOBENWEYL dans l'ouvrage intitulé "Method der Organischen Chemie" (Méthode de la Chimie Organique) édité par E. Wunsch, vol. 15- I et II., THIEME, Stuttgart 1974.

Cette méthode de synthèse consiste à condenser successivement deux-à-deux les aminoacyles successifs dans l'ordre requis, ou à condamner des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodimide, tels que par exemple la 1-éthyl-3-(3-diméthyl-aminopropyl)-carbodiimide. Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptidique synthesis" (J. Am. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherchée, à partir du résidu amino-acyle C-terminal sur la fonction amine déprotégée du premier acide aminé C-terminal fixé sur la chaîne. De préférence, la fonction carboxyle de ce deuxième acide aminé est activée, par exemple par la dicyclohexylcarbodiimide, et par la fonction amine est protégée, par exemple par le t-butyloxycarbonyle.

On obtient ainsi la première partie de la chaîne peptidique recherchée, qui comporte deux acides aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acides aminés constituant la chaîne peptidique et on détache le peptide de la résine par exemple à l'aide d'acide fluorhydrique.

L'invention concerne également les oligomères hydrosolubles des peptides monomères sus-indiqués. L'oligomérisation peut provoquer un accroissement de l'immunogénicité des peptides monomères selon l'invention. Sans qu'une telle indication chiffrée puisse être considérée comme limitative, on mentionnera néanmoins que ces oligomères peuvent, par exemple, contenir de 2 à 10 unités monomères.

Les unités monomères entrant dans cet oligomère sont soit toutes constituées par le polypeptide de séquence I ou par le polypeptide de séquence II, III ou IV.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode d'oligomérisation ou de polymérisation du monomère consiste dans la réaction de celui-ci avec un agent de réticulation tel que le glytaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro- bifonctionnels.

On peut également pour la production de molécules comportant un ou plusieurs motifs de 64 acides aminés tels que définis ci-dessus, avoir recours à des techniques du génie génétique mettant en oeuvre des micro-organismes transformés par un acide nucléique déterminé comprenant des séquences nucléotidiques appropriées correspondantes.

A ce titre, l'invention concerne également des acides nucléiques contenant une ou plusieurs de ces séquences comprenant chacune 64 triplets du genre sus-indiqué.

L'invention concerne encore les conjugués obtenus par couplage covalent des peptides selon l'invention (ou des susdits oligomères) à des molécules porteuses (naturelles ou synthétiques), physiologiquement acceptables et non toxiques, par l'intermédiaire de groupements réactifs complémentaires respectivement portés par la molécule porteuse et le peptide. Des exemples de groupements appropriés sont illustrés dans ce qui suit :

A titre d'exemple de molécules porteuses ou supports macromoléculaires entrant dans la constitution des conjugués selon l'invention, on mentionnera des protéines naturelles, telles que l'anatoxine tétanique, l'ovalbulmine, des sérums albumines, des hémocyamines, etc...

A titre de supports macromoléculaires synthétiques, on mentionnera par exemple des polylysines ou des poly (D-L-alanine)-poly(L-lysine).

La littérature mentionne d'autres types de supports macromoléculaires susceptibles d'être utilisés, lesquels présentent en général un poids moléculaire supérieur à 20.000.

Pour synthétiser les conjugués selon l'invention, on peut avoir recours à des procédés connus en soi, tels que celui décrit par FRANTS et ROBERTSON dans Infect. and Immunity, 33, 193-198 (1981), ou celui décrit dans Applied and Environmental Microbiology, (octobre 1981), vol. 42, n° 4, 611-614 par P.E. KAUFFMAN en utilisant le peptide et la molécule porteuse appropriée.

Dans la pratique, on utilisera avantageusement comme agent de couplage les composés suivants, cités à titre non limitatif : aldéhyde glutarique, chloroformiate d'éthyle, carbodiimides hydrosolubles [N-éthyl-N'(3-diméthylamino-propyl) carbodiimide, HCl], diisolcyanates, bis-diazobenzidine, di- et trichloro-s-triazines, bromures de cyanogène, ainsi que les agents de couplage mentionnés dans Scand. J. Immunol.n (1978), vol. 8, 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives de molécules supports. Avantageusement, il s'agit des fonctions carboxyle et amine, lesquelles peuvent donner lieu à une réaction de couplage en présence d'une agent de couplage du genre de ceux utilisés dans la synthèse des protéines, par exemple, le 1-éthyl-3 (3-diméthylaminopropyl) -carbodiimide, le N-hydroxybenzotriazole, etc... On peut encore avoir recours à la glutaraldéhyde, notamment lorsqu'il s'agit de relier entre eux des groupes aminés respectivement portés par le peptide et la molécules support.

Un groupe de molécules préférées selon l'invention est constitué de celles possédant une conformation en hélice a, cette dernière renforçant les propriétés antigéniques, et immunogéniques desdites molécules. De telles molécules possédant une conformation en hélice a ont été mise en évidence par dichroisme circulataire dans trifluoroethanol, ou en solution aqueuse.

Les molécules selon l'invention possèdent des propriétés antigéniques caractéristiques de l'antigène 48 Kd du merozoïte spécifique du stade enythrocytaire du développement de P. falciparum, et présentant les caractéristiques particulières décrits plus haut.

En effet, comme il le sera plus particulièrement décrit à l'aide d'exemples de molécules selon l'invention dans la description détaillée qui suit, les molécules selon l'invention réagissent spécifiquement avec les anticorps anti protéine 48 Kd d'isotype majoritaire $IgG_2$ ou IgM chez les patients sensibles à l'infection, et d'isotype majoritaire IgG1 ou IgG3 chez les sujets prémunis.

Ces molécules selon l'invention sont capables de déclencher in vivo la synthèse d'immunoglobulines spécifiques, et susceptibles d'induire in vivo la neutralisation du mérozoïte présent dans le sang, son processus dans les monocytes et l'inactivation du développement intraéthrocytaire du P. falciparum à la suite d'une interaction entre les monocytes et les parasites libres extra-érythrocytaires ou merozoïtes par l'intermédiaire d'un anticorps cytophile par liaison du fragment Fc de l'immunoglobuline au récepteur γ du monocyte.

La Figure 1 correspond à la mise en évidence de la protéine parasitaire de 48000 (indiquée par les flèches) en immunoblot. La réactivité du sérum de souris immunisée avec DG210 est étudiée en immunotransfert sur les antigènes de stades sanguins de P. falciparum extraits en SDS (ser anti-R210) ou en Triton-X114 phase détergent (A) et phase aqueuse (B). La réactivité des sérums humains est étudiée sur les extraits SDS en révélant les isotypes IgG1 (1), IgG2 (2), IgG3 (3) et IgG4 (4). SHI : sérum hyperimmun.

Dans l'exemple suivant, comme dans toutes les expériences décrites dans la présente description, les immunoglobulines de plasma humain sont obtenues par la méthode décrite par A. SABCHAREON et coll., J. Trop. Med. Hyg. 1991, 45(3):297). Le test ADCI est décrit plus haut.

Dans l'exemple suivant sont comparés les index spécifiques d'inhibition (I.S.) obtenus aussi bien avec des sérums de souris immunisées avec le peptide III, qu'avec les anticorps humains immuns purifiés à l'aide d'une colonne d'affinité porteuse du peptide III (technique décrite dans OKAZI et coll.). Les uns et les autres sont capables de reconnaître la protéine 48 Kd aussi bien en immunofluorescence indirecte que par des tests en Western blott, et cela dans les mêmes conditions que précédemment (IgG2 de patients sensibles et IgG1 et IgG 3de patients prémunis). Enfin, les anticorps immunopurifiés, comme les anticorps induits par injection du peptide III à la souris, éprouvés en tests ADCI, confirment qu'ils sont capables d'induire l'inactivation du parasite par l'intermédiaire des monocytes.

Le tableau I suivant résume les résultats à l'appui de ces observations.

| | Anticorps | Index d'inhibition spécifique (%) |
|---|---|---|
| Témoins(+) | Pshi | 60 |
| | shi1 | 77 |
| | shi2 | 66 |
| Témoins(-) | spi | 0 |
| | anti-βgal | -18 |
| Test | anti-DG210 | 45 |
| | anti-DG328 | -13 |
| | anti-DG414 | 4 |
| | anti-210B1 | 72 |
| | B2 | 80 |
| compétitions | Pshi+Acm245 | 20 |
| | Pshi+spi | 23 |

Tableau 1

dans lesquels Pshi représente un pool de sérum hyperimmun, shi 1 et 2 des sérums hyperimmuns de deux donneurs différents, spi et anti-βgal, des témoins issus de sérum après une primo invasion et un témoin anti-βgal; anti-DG210 sont des anticorps purifiés contre le peptide I, anti-210B (1) sont des anticorps humains purifiés contre le peptide III anti-210B (2) sont les anticorps induits chez la souris et les anti-R328 et R414 sont des anticorps purifiés contre des peptides en provenance d'autres clones.

L'index spécifique d'inhibition est celui mesuré par la technique d'ADCI.

Les molécules selon l'invention sont donc capables d'induire la synthèse d'anticorps d'une classe capable de coopérer avec des monocytes.

Les protéines et les peptides de l'invention ne sont pas limités à ceux particulièrement décrits ci-dessus.

L'invention vise tous les peptides ou polypeptides naturels, de recombinaison génétique ou de synthèse qui présentent les mêmes propriétés d'être susceptibles d'induire des mécanismes de défense immunitaire développés et caractéristiques des sujets protégés par le paludisme.

Par cet aspect, l'invention vise particulièrement des épitopes de la protéine 48 Kd, différents des polypeptides II, III, et IV ci-dessus. En effet, nous avons pu montrer que les immunoglobulines de certains individus réagissent avec un épitope de la protéine 48 Kd en Western blott, alors que ces mêmes immunoglobulines ne reconnaissent pas l'antigène exprimé par le clone DG210.

L'invention concerne également les anticorps, polyclonaux ou monoclonaux présentant la caractéristique de reconnaître les molécules de l'invention et de coopérer avec les monocytes, et susceptibles d'être utilisés dans des compositions pharmaceutiques pour protéger par immunothérapie passive, des sujets infectés et présentant ou pouvant présenter les symptômes de la maladie.

Les anticorps monoclonaux peuvent être produits par la technique des hybridomes, suivant les techniques classiques comprenant :

- la fusion d'une cellule myelomateuse avec des cellules spléniques d'un animal préalablement immunisé avec l'un des antigènes selon l'invention,
- la culture des hybridomes formés par la fusion des cellules précédentes et,
- la sélection de ceux des hybridomes capables de former des anticorps monoclonaux reconnaissant l'antigène utilisé pour l'immunisation des animaux.

Les animaux choisis pour l'immunisation peuvent être par exemple des souris.

Parmi ces anticorps monoclonaux, on sélectionnera avantageusement les anticorps monoclonaux cytophiles, c'est à dire ceux dont le fragment Fc est capable de se fixer sur le récepteur Fc des monocytes humains.

Une autre technique de production d'anticorps peut permettre de réaliser in vitro des anticorps monoclonaux humains. Pour ce faire, on utilise des lymphocytes B immortalisés par exemple avec le virus d'Epstein Barr. Ces lymphocytes peuvent être prélevés chez une personne ayant été infectée par P. falciparum. Dans ce cas, ils permettent la production d'anticorps monoclonaux contre plusieurs antigènes sans avoir recours à une stimulation in vitro par de nouveaux antigènes.

Une autre possibilité consiste à fusionner des lymphocytes B immortalisés de la façon décrite plus haut, avec des lymphocytes B humains préalablement stimulés in vitro avec un antigène selon l'invention contre lequel on cherche à former des anticorps monoclonaux, dans des conditions de culture permettant la stimulation des lymphocytes.

On se reportera avantageusement à la technique décrite par Desgranges C. et al (1987, J of Virological Methods, vol 16, p281-292) pour la préparation des anticorps monoclonaux humains de l'invention.

Il est également envisagé dans le cadre de l'invention de produire des anticorps monoclonaux humains par recombinaison génétique en réalisant une transfection in vitro du gène codant pour la partie variable de l'anticorps, dans des vecteurs infectant des bactéries, dans des conditions permettant l'expression d'une immunoglobuline humaine.

Enfin, la présente invention concerne tout type d'anticorps monoclonal chimérique, hybride, ou encore tout fragment d'anticorps monoclonal ou polyclonal de type Fab ou Fab'2, et répondant aux mêmes caractéristiques d'affinité pour les épitopes de la protéine 48 Kd ou les peptides I, II et III, IV ci-dessous.

Des anticorps monoclonaux préférés selon l'invention sont des anticorps humains de classe IgG, ou $IgG_3$, ou des anticorps obtenus chez l'animal et ayant des propriétés cytophiles chez l'homme, dirigés contre l'un ou plusieurs des antigènes dont la séquence a été décrite plus haut.

L'invention concerne également un procédé pour le suivi de la vaccination de patient contre l'infection contre P. falciparum, à partir d'un échantillon biologique tel que le sang, caractérisé en ce qu'il comprend :

- la mise en contact de l'échantillon biologique susceptible de contenir des anticorps protecteurs contre P. falciparum, avec au moins un antigène selon l'invention,
- la détection de la réaction antigène-anticorps.

Pour la réalisation de cette méthode de détection in vitro, les antigènes selon l'invention sont avantageusement marqués à l'aide d'un marqueur radioactif, d'un marqueur enzymatique ou fluorescent ou encore d'un marqueur de type marqueur physique.

L'invention vise également des kits pour la détection in vitro de la présence d'anticorps dirigés contre les antigènes de l'invention, caractérisés en ce qu'ils contiennent :

- une composition antigénique comprenant au moins un antigène selon l'invention,
- des réactifs nécessaires à la réalisation de la réaction immunologique entre les susdits antigènes et les anticorps éventuellement pris dans l'échantillon biologique,
- des réactifs permettant la détection du complexe antigène-anticorps produit par la réaction immunologique.

Ces réactifs sont par exemple porteurs d'un marqueur ou susceptibles d'être reconnus par un réactif marqué.

II- Isolement du clone DG 210 :

a) construction de la banque

Une banque de DNA (ADN) génomique a été construite dans le bactériophage d'expression λgt11, en utilisant l'ADN génomique du clone Tak 9-96 de P. falciparum (ref clone Tak 9-96 : Science 212, 137; 1981) suivant le protocole décrit en détail dans la demande de brevet EP du 9 février 1987 publiée sous le numéro 0343186.

En bref, l'ADN a été découpé par la DNAase I, en présence d'ions $Mn^{2+}$, méthylé par la méthylase EcoRI pour protéger les sites EcoRI naturels, puis réparé par l'ADN polymérase du bactériophage T4 et l'ADN ligase d'EcoRI. Des "linkers" oligomères synthétiques) EcoRI ont été ligaturés aux fragments de DNA de P. falciparum et les sites artificiels ainsi rajoutés ont été libérés par coupure avec l'enzyme EcoRI. Les fragments ont été purifiés sur gradient de sucrose et ligaturés avec le DNA du vecteur λgt11 convenablement préparé (c'est à dire coupé par EcoRI et déphosphorylé - vendu par Promega Biotec). L'ADN a été encapsidé in vitro dans des particules virales. Les bactériophages issus de ce procédé constituent une banque d'ADN génomique.

b) Criblage immunologique de la banque

Les détails techniques de criblage sont donnés dans le texte de la demande de brevet 0343186. Parmi une série d'anticorps monoclonaux (AcM) utilisés antérieurement, l'AcM 245 (Soulier et coll., Revue Française de Transfusion et Immunohématologie, Tome XXV, n° 4, 1982, page 373) de classe IgM, une classe d'anticorps incapable de coopérer avec les monocytes, est le seul qui se soit avéré capable d'entrer en compétition avec les anticorps polyclonaux de sujet immun actif dans le test d'ADCI, c'est à dire de réduire sensiblement l'effet inhibiteur de ces anticorps en ADCI, suggérant que l'épitope cible de ces anticorps capables de coopérer avec les monocytes et de cet AcM 245 est identique. C'est cet anticorps qui a été utilisé pour l'isolement du gène par criblage d'une bande d'ADN génomique clonée dans le vecteur d'expression λgt11.

Un criblage direct par réaction antigène/anticorps avec les protéines synthétisées par les clones de la banque s'est avéré infructueux. Dans la mesure où cet AcM est capable d'entrer en compétition avec d'autres anticorps pour un épitope porté par la protéine parasitaire, une autre méthode de criblage a alors été utilisée.

Les antigènes recombinants ont été criblés par un test de compétition en immunofluorescence indirecte. L'anticorps monoclonal AcM 243 en présence du mérozoïte a été incubé avec chacun des antigènes recombinants (surnageant des différents clones de la bande génomique) et l'inhibition de la fixation de l'anticorps sur le parasite a été mesuré par immunofluorescence indirecte (technique décrite dans H. BOUHAROUN-TAYOUN et al, 1990, J. Exp. Med. 172: 1633-1641).

Six antigènes se sont avérés positifs, c'est à dire bloquants, et ont été étudiés en détail. Ces six protéines antigènes ainsi sélectionnées ont été fixées sur des résines afin de réaliser des purifications par affinité des anticorps polyclonaux issus de sérums humains immuns, selon la technique décrite par OKAZI et Coll. Ces immunoglobulines ainsi purifiées ont été étudiées. Parmi celles-ci, l'une obtenue par fixation sur la protéine synthétisée par le clone DG 210 reconnaît en Western blott le doublet de 48 Kd lequel doublet apparaît être identique à celui reconnu par les classes d'IgG cytophiles trouvées chez les sujets adultes dans un état de résistance clinique à la maladie et par les classes non cytophiles des individus sensibles. En revanche, il est différent de l'antigène MSA2, un antigène de surface du mérozoite qui sur le même gène apparaît comme un polypeptide d'un poids moléculaire supérieur (figures). Les résultats du tableau I montrent que les anticorps isolés par immunoaffinité sur la protéine sécrétée par le clone DG 210 sont capables de promouvoir in vitro l'effet inhibiteur de la croissance du trophozoïte induit par le monocytes, par la technique ADCI.

Le clone DG 210 a été déposé à la CNCM le 19 octobre 1992 sous le numéro n° I-1270.

- Caractérisation de la protéine synthétisée par le clone Dg 210.

Les anticorps humains immunoabsorbés sur cette protéine comme ceux produits chez la souris par immunisation avec le clone DG210 montrent en immunofluorescence indirecte un image en grappe dessinant le pourtour des mérozoites, au sein des schizontes intra-érythrocytaires mûrs. Cette indication que la molécule est localisée au niveau de la membrane des mérozoites a été confirmée, d'une part par extraction avec un détergent non ionique, le triton X 114, à partir de mérozoites purifiés et détection de la protéine dans la phase "détergent" soluble ; d'autre part par action de la phospholipase C de bacillus aureus, cette enzyme libérant la protéine à partir d'une préparation de mérozoites purifiés ce qui indique ainsi que celle-ci est ancrée par un groupement phosphatidyl-inositol ; enfin par révélation de la localisation des anticorps en microscopie électronique à l'aide d'un deuxième anticorps marqué à l'or colloïdal : ces anticorps sont principalement dirigés contre un Ag situé à la surface des mérozoïtes de P. falciparum.

Ces résultats confirment que l'antigène capable de stimuler le mécanisme de cytotoxicité dépendante d'anticorps (ADCI) est situé en surface de la forme extracellulaire du parasite, le mérozoite. En outre les anticorps obtenus par immunoaffinité sur le produit recombinant du clone Dg 210 ont un pouvoir très fortement inhibiteur de la croissance du P. falciparum dans le test d'ADCI alors que ces mêmes anticorps n'ont aucun effet sur l'infection du globule rouge par le mérozoite. Les anticorps-contrôle préparés de la même façon avec d'autres protéines recombinantes-contrôle incluant le MSA2 et le RESA n'ont aucun effet inhibiteur ni directement ni dans les essais ADCI (figure). Les résultats se retrouvent dans trois expériences séparées mettant en jeu trois isolats différents d'anticorps. Deux de ces résultats sont montrés dans la figure 1.

Ces résultats sont confirmés par des observations complémentaires. La distribution isotypique des anticorps dirigés contre la protéine recombinante issue du clone Dg 210 présente les caractéristiques suivantes. Des isotypes IgG2 sont trouvés de façon beaucoup plus abondante dans les patients non protégés alors que la protéine est reconnue de façon préférentielle par des IgG1 et IgG3 cytophiles dans le sang de sujets protégés. Ainsi les épitopes contenus dans la protéine recombinante du clone Dg210 ont toutes les caractéristiques recherchées pour une protéine à effet vaccinant, à savoir qu'ils pourraient induire in vivo des anticorps non-cytophiles dans des sujets non protégés, qui au contraire sont cytophiles dans des sujets protégés et ainsi être capables d'induire la réaction d'ADCI in vivo.

Enfin l'étude de la réponse lymphoproliférative de 70 sujets exposés au paludisme (au Sénégal et à Madagascar) révèle que les peptides II, III et IV définissent des épitopes reconnus par les lymphocytes T.

Une forte prévalence de réponses lymphoprolifératives (>50% de l'effectif étudié) a été observée chez ces sujets exposés à la maladie.

- Séquençage et caractérisation du génome du clone DG 210.

Le génome du clone DG210 a une longueur de 1300 paires de base. La taille a pu en être déterminée par la mise en oeuvre de la méthode décrite par Mc Cutchans (Mc Cutchan et al (1984), Science 225: 625-627). Brièvement, le génome est digéré par l'endonucléase du haricot (Mung Bean), les fragments de restriction sont ensuite hybridés avec la sonde DG210 marquée au Phosphore 32, et révélés en autoradiographie selon des techniques bien connues de l'homme du métier.

L'étude en "Northern Blott" de ces mêmes fragments et révélée par la même sonde radioactive, confirme que le gène est exprimé pendant la phase erythrocytaire du cycle du parasite.

L'analyse de la séquence des 192 paires de bases de l'insert a été réalisée par la méthode de Sanger et al (PNAS, 74:5463, 1977) dite méthode de "2 desoxyterminaison".

L'invention concerne encore les acides nucléiques recombinants contenant l'une au moins des séquences polypeptidiques I, II, III ou IV ou une combinaison de celles-ci ainsi que les micro-organismes, notamment les bactéries E. coli transformées par ces acides nucléiques recombinants et capables d'exprimer lesdits polypeptides.

L'invention concerne ces séquences d'acides nucléiques ou des séquences équivalentes qui peuvent être synthétisées et qui codent pour les mêmes acides aminés.

Il apparaîtra immédiatement à l'homme du métier que dans ces séquences, certains des nucléotides peuvent être remplacés par d'autres en raison de la dégénérescence du code génétique sans que pour autant les peptides codés ne soient modifiés. Toutes ces séquences nucléotidiques, ainsi que celles qui codent pour des polypeptides qui diffèrent des précédents par un ou plusieurs acides aminés sans que leur activité immunogénique propre ne soit modifiée de façon semblable, font partie de l'invention. Il en va naturellement de même des séquences nucléotidiques qui peuvent être reconstituées et qui sont capables de coder pour des oligomères tels qu'ils ont été définis plus haut. Les motifs monomères sont liés directement bout à bout ou par l'intermédiaire de séquences peptidiques sans effet sur les propriétés immunogéniques des oligomères ainsi formés.

Enfin, l'invention concerne les vecteurs modifiés par ces séquences, ces vecteurs étant naturellement pourvus de régulation et de terminaison précédant et suivant les séquences nucléiques sus-indiquées, qui permettront l'expression de ces dernières dans des organismes cellulaires compétents.

Parmi les séquences nucléotidiques qui codent pour les peptides caractéristiques qui ont été définis ci-dessus, on mentionnera celles qui sont caractérisées par les séquences de triplets qui suivent, ces séquences correspondant en particulier pour la première au peptide I et pour les trois suivantes aux peptides II, III et IV dont les formules ont été précédemment indiquées.

**CAT GAA AGG GCA AAA AAT GCT TAT CAA AAA**
**GCA AAC CAA GCT GTT TTA AAA GCA AAA GAA**
**GCT TCT AGT TAT GAT TAT ATT TTA GGT TGG** **(1)**
**GAA TTT GGA GGA GGC GTT CCA GAA CAC AAA**
**AAA GAA GAA AAT ATG TTA TCA CAT TTA TAT**
**GTT TCT TCA AAG GAT AAG GAA AAT ATA TCT**
**AAG GAA AAT GAG**

**CAT GAA AGG GCA AAA AAT GCT TAT CAA AAA**
**GCA AAC CAA GCT GTT TTA AAA GCA AAA GAA** **(2)**
**GCT TCT AGT TAT GAT**

**GCA AAA GAA GCT TCT AGT TAT GAT TAT ATT**
**TTA GGT TGG GAA TTT GGA GGA GGC GTT CCA** **(3)**
**GAA CAC AAA AAA GAA GAA AAT**

CCA GAA CAC AAA AAA GAA GAA AAT ATG TTA
TCA CAT TTA TAT GTT TCT TCA AAG GAT AAG (4)
GAA AAT ATA TCT AAG GAA AAT GAG

Des bactéries hébergeant les susdits clones DG210 ont été déposées à la Collection Nationale des Cultures de Microorganismes de l'Institut Pasteur de Paris (CNCM), le 19 octobre 1992 sous le numéro I-1270.

L'invention a aussi pour objet des amorces d'ADN ou d'ARN utilisables par exemple dans le cadre de la synthèse de séquences nucléotidiques éventuellement suivie de la synthèse polypeptidique selon l'invention par la technique de la PCR (Polymerase Chain Reaction) telle que décrite dans les brevets américains n° 4683212 et 4683195 et de la demande de brevet européen n 200362. Une description du procédé utilisé ici se trouve dans la demande de brevet PCT n° FR 91/00639, pages 28 à 30.

Les peptides de l'invention peuvent encore être préparés par les techniques classiques dans le domaine de la synthèse peptidique. Cette synthèse peut être réalisée en solution homogène ou en phase solide tel que décrit plus haut par la technique de HOBENWEYL ou de MERRYFIELD.

III- Etude du polymorphisme du gène, et des épitopes définis par le clone DG210 :

Un frein majeur à l'obtention d'un vaccin efficace est, outre la complexité du cycle du parasite, sa diversité antigénique, et le haut degré de polymorphisme d'une souche à l'autre.

La conservation du gène et des épitopes définis dans le clone DG210 a été étudiée par plusieurs techniques dans une série d'isolats de plasmodiae.

En utilisant les nucléotides suivants comme amorce:

GAA AGG GCA AAA AAT GCT TAT (5)

ou

TAA AAG GAA TCT ATA TAA AAG (6)

les fragments d'ADN de deux cultures de souches cultivées de P. falciparum africaines de 4 isolats Thai et de 29 isolats africains ont pu être amplifiées par la technique PCR.

Le gène correspondant était présent partout, avec aucun polymorphisme apparent de taille, alors qu'une expérience similaire par la même technique PCR avec les amorces des régions MSA1 et MSA2 n'a pu mettre en évidence ce résultat.

De la même façon, le screening des protéines et peptides par Western Blott, préparés à partir de 6 isolats Thai ou africains avec des anticorps purifiés à partir d'une colonne d'affinité avec le peptide 210 comme ligand, ont permis de révéler le doublet à 48 kD dans tous les variants, sans modification de poids moléculaire d'un isolat à l'autre.

Enfin, 10 isolats du Congo ont été étudiés par immunofluorescence indirecte par la même technique que plus haut ont tous été positifs, et tous les parasites de chacun des isolats ont été marqués par les anticorps purifiés par affinité.

Tout semble donc indiquer l'absence de polymorphisme antigénique, au moins dans la région de la molécule portant l'épitope B, de même que la conservation de la taille de cette protéine d'un isolat à l'autre.

Ces résultats confirment ceux obtenus en ADCI, et plus particulièrement dans les tests de compétition dans lesquels les anticorps non cytophiles obtenus après une attaque primaire par le parasite sont d'excellents compétiteurs des anticorps cytophiles des adultes prémunis.

Dans la mesure où les anticorps non cytophiles obtenus après l'attaque primaire correspondent à un seul isolat, et les adultes prémunis sont protégés contre l'infection d'un grand nombre d'isolats polymorphes (qui, de plus, ont été isolés dans les expériences de compétition), on est en droit de conclure que les épitopes concernés dans les expériences de compétition sont représentatifs de régions conservées, non polymorphes.

Les polypeptides et protéines de l'invention sont donc caractérisés par un large spectre d'action en tant que composition vaccinante.

**Revendications**

1. Molécule, peptide ou polypeptide purifiés **caractérisés en ce qu'**ils sont :

   - reconnus principalement par des anticorps cytophiles de sujets prémunis contre l'infection par les *Plasmodia* et reconnus par des anticorps non cytophiles de sujets sensibles à l'infection par les *Plasmodia,* et
   - susceptibles d'inhiber la fixation sur un mérozoïte, d'un anticorps lui-même capable de réduire sensiblement l'effet d'inhibition cellulaire anticorps-dépendante (ADCI) des anticorps de sujets prémunis contre l'infection contre les *Plasmodia.*

2. Molécule, peptide ou polypeptide selon la revendication 1, **caractérisés en ce qu'**ils présentent en outre un caractère peu polymorphe au sein des *Plasmodia.*

3. Molécule selon la revendication 1 ou la revendication 2, **caractérisée en ce qu'**elle est fixée à un support.

4. Composition immunogène **caractérisée par** l'association d'une molécule conforme à l'une quelconque des revendications 1 à 3 avec un véhicule pharmaceutiquement acceptable.

5. Composition immunogène selon la revendication 4, **caractérisée en ce qu'**elle induit la synthèse d'anticorps à caractère cytophile contre ledit immunogène, lesquels anticorps sont capables d'induire une réaction cytotoxique vis-à-vis des stades érythrocytaires du parasite ou l'un de ses précurseurs.

6. Composition de vaccin dirigée contre le paludisme comprenant entre autres principes immunogènes, une molécule conforme à l'une quelconque des revendications 1 à 3.

7. Anticorps polyclonaux ou monoclonaux **caractérisés en ce qu'**ils reconnaissent les molécules, protéines ou peptides selon l'une quelconque des revendications 1 à 3.

8. Anticorps selon la revendication 7, **caractérisés en ce qu'**ils sont cytophiles.

9. Anticorps selon la revendication 7 ou la revendication 8, **caractérisés en ce qu'**ils sont susceptibles de déclencher une réaction cytotoxique vis-à-vis des stades érythrocytaires du parasite ou de l'un de ses précurseurs.

10. Anticorps polyclonaux ou monoclonaux selon l'une quelconque des revendications 7 à 9, **caractérisés en ce que** cette réaction est médiée par les monocytes ou macrophages.

11. Anticorps hybrides ou chimères d'anticorps selon l'une quelconque des revendications 7 à 10 et présentant les mêmes caractéristiques que cesdits anticorps, à savoir induisant une réaction cytotoxique contre des stades érythrocytaires du parasite ou de l'un de ses précurseurs.

12. Composition **caractérisée par** l'association d'anticorps selon l'une quelconque des revendications 7 à 11 avec un véhicule pharmaceutiquement acceptable.

13. Composition pour immunothérapie passive de sujets sensibles à l'infection par un parasite de la famille des Plasmodiae, **caractérisée par le fait qu'**elle contient des anticorps selon l'une quelconque des revendications 7 à 11.

14. Utilisation d'anticorps cytophiles selon l'une quelconque des revendications 7 à 11, pour la préparation d'une composition pharmaceutique destinée à la prévention, au diagnostic, et/ou au traitement des infections parasitaires dues à des *Plasmodia.*

15. Méthode de diagnostic *in vitro* du paludisme chez un individu susceptible d'être infecté par un plasmodium qui comprend la mise en contact d'un tissu ou d'un fluide biologique prélevé chez un individu, avec une composition polypeptidique selon l'une quelconque des revendications 1 à 3, dans des conditions permettant une réaction immunologique *in vitro* entre ladite composition polypeptidique et les anticorps éventuellement présents dans le tissu ou le fluide biologique, et la détection *in vitro* du complexe antigène-anticorps éventuellement formé.

16. Méthode de diagnostic selon la revendication 15, **caractérisée par le fait qu'**elle s'applique au diagnostic d'un large spectre d'infections par un plasmodium.

17. Coffret ou kit pour le diagnostic *in vitro* du paludisme chez un individu susceptible d'être infecté par un plasmodium qui comprend :

    - une composition polypeptidique selon l'une quelconque des revendications 1 à 3,
    - les réactifs pour la constitution du milieu propice à la réalisation de la réaction antigène-anticorps,
    - les réactifs permettant la détection du complexe formé ; de tels réactifs peuvent également porter un marqueur, ou sont susceptibles d'être à leur tour reconnus par un réactif marqué, plus particulièrement dans le cas où la composition polypeptidique sus-mentionnée n'est pas marquée.

18. Coffret ou kit pour le diagnostic *in vitro* du paludisme chez un individu susceptible d'être infecté par un plasmodium qui comprend :

    - une composition d'anticorps selon l'une quelconque des revendications 7 à 11,
    - les réactifs pour la constitution du milieu propice à la réalisation de la réaction antigène-anticorps,
    - les réactifs permettant la détection du complexe formé ; de tels réactifs peuvent porter un marqueur, ou sont susceptibles d'être à leur tour reconnus par un réactif marqué, plus particulièrement dans le cas où la composition d'anticorps sus-mentionnée n'est pas marquée.

19. Complexe comprenant une molécule, peptide ou polypeptide selon l'une quelconque des revendications 1 à 3, un anticorps selon l'une quelconque des revendications 7 à 11, et un monocyte.

20. Complexe comprenant une molécule selon la revendication 1 ou la revendication 2 et un monocyte, **caractérisé en ce que** ladite molécule est une protéine du mérozoïte de Plasmodium *falciparum* et **en ce que** la liaison entre ladite molécule et ledit monocyte est effectuée au moyen d'un anticorps cytophile.

**21.** Complexe selon la revendication 20, **caractérisé en ce que** l'anticorps cytophile est une immunoglobuline de classe IgG1.

**22.** Complexe selon la revendication 20, **caractérisé en ce que** l'anticorps cytophile est une immunoglobuline de classe IgG3.

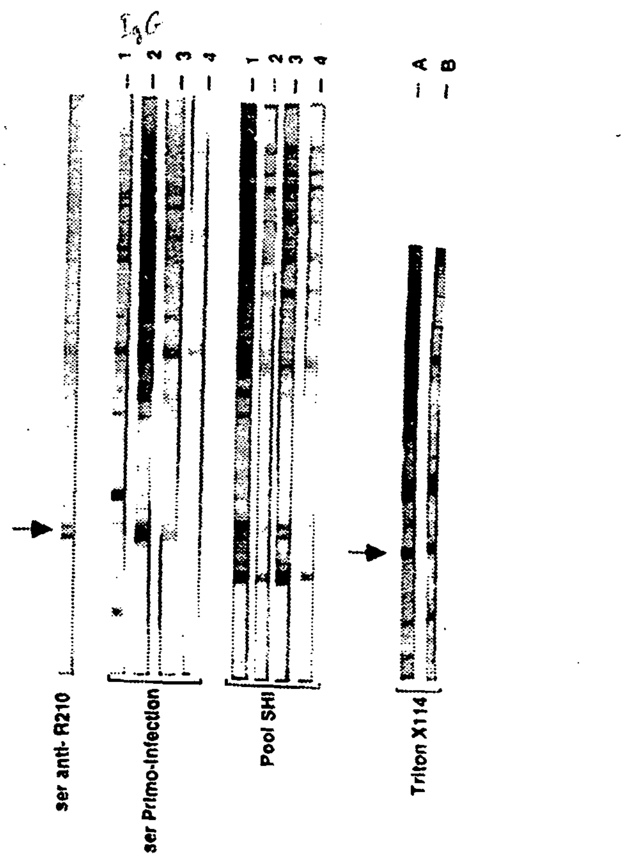
IgG
1
2
3
4
1
2
3
4
A
B
ser anti- R210
ser Primo-Infection
Pool SHI
Triton X114

Figure 1